# EUROPEAN PATENT SPECIFICATION

(11) **EP 4 106 709 B1**
(45) Date of publication and mention of the grant of the patent: **18.12.2024**
(21) Application number: 21707792.4
(22) Date of filing: 17.02.2021
(51) Int. Cl.: A61H 9/00

(54) **STRUCTURE FOR THERAPEUTIC APPLICATIONS**
STRUKTUR FÜR THERAPEUTISCHE ANWENDUNGEN
STRUCTURE POUR APPLICATIONS THÉRAPEUTIQUES

(30) Priority: 18.02.2020 CH 1762020
(43) Date of publication of application: 28.12.2022
(73) Proprietor: JK-Holding GmbH, 53578 Windhagen (DE)
(72) Inventor: GERSTENMEIER, Jürgen, 56567 Neuwied (DE)
(74) Representative: IPrime Rentsch Kaelin AG
(86) International application number: PCT/IB2021/051328
(87) International publication number: WO 2021/165845

(56) References cited:
- WO-A1-2007/141680
- WO-A1-2012/055029
- WO-A1-2019/076459
- WO-A2-2010/101633
- CN-A- 109 925 583
- JP-A- 2004 208 746
- JP-A- 2007 325 712
- US-A1- 2015 320 352
- US-A1- 2019 290 132

## Description

The present invention relates to a structure for therapeutic applications, in particular, to a layered structure for wellness, relaxation, or fitness, all according to the preambles of the independent claims.

### Technological background

Therapeutic applications are offered as treatments to patients for medical or relaxation purposes. Massages are ideal therapies for patients suffering from stress, muscular tension and back pain. Therapeutic whole body or partial body massages using targeted forces in order to relax the muscles to soothe stiffness or cramps and to relive back and joint pain, muscle tension, muscular imbalances, stress-related muscular tension, or cervical migraine. Beneficial warmth may stimulate the entire metabolism. Help can be achieved for example by dry water massages.

Dry water massage or other water devices are known. These are devices that usually perform effective massage treatment automatically. Separated from the water by a rubber mat, a patient lies on warm water. From nozzles, which can be moved, water jets are pumped from below against the rubber mat on which the patient is lying. A treatment with a certain pressure and a treatment time of approximately 1 min to 45 min can be set individually via a keyboard or via chip cards with fixed or pre-programmed programs.

Fixed massage functions, defined massage areas and massage courses can be defined and called up accordingly. Storage on a chip card is possible. The water temperature can usually be adjusted in steps from approx. 20°C to 40°C.

During an application, the warmth of the water radiates into deep tissue layers and promotes the blood circulation of the patient. The power of the water activates the metabolism, loosens the muscles, releases tension and hardenings. An advantage of this special therapy is that it is carried out without stressing the circulation or after-sweating.

The concept of dry water massage can be used for therapy and prevention. The following therapeutic demands are made on such devices: pain relief, muscle relaxation, elimination of muscular imbalances, increase or decrease of muscle tone, local increase of blood circulation and metabolism, loosening of subcutaneous cell tissue, venous and lymphatic decongestion, reflective relief of pain, loosening of scar tissue, activation or attenuation of the vegetative nervous system, increase of the efficiency of subsequent therapeutic measures.

These requirements are achieved by the massage effect of the water jets, which produce the massage effect from one or more jets under the patient support, and by the warming effect.

The massage effect of the water and the heat transfer via the patient support to the patient represents a considerable enrichment in the treatment of back pain patients.

Compared to underwater massage, overwater or dry water massage systems or devices have the advantage that the patient does not have to go into the water and therefore does not have to remove bandages, gauze bandages, etc.

The patient support is provided by a mat made of a coextruded natural rubber material. There are problems with the surface quality and there are certain limits to the strength. In general, there is a need for advancements in this field of technology.

JP2004208746 A discloses a device according to the preamble of claim 1.

### Summary of the Invention

The invention is set out in the appended set of claims. It is an object of the invention to provide a structure or support for therapeutic applications. The therapeutic applications and methods are not part of the present invention. It is a further object of the invention to provide a mat with improved properties for therapeutic applications. It is an object of the invention to provide an improved hydromassage device.

At least one of these objects is achieved by the features according to the independent claims. Further particular embodiments are indicated in the dependent claims and the description.

A first aspect of the invention relates to a structure for therapeutic applications that comprises at least one layer with sensor elements and may comprise heating elements. A first force affects the layer from one side and a second force affects the layer from the other side. Different forces are applied from the two sides.

In a further aspect of the invention a mat is provided that comprises the structure. The mat can be used for various therapeutic applications (not part of the present invention), for example, for wellness, relaxation, sport or fitness applications.

Another aspect of the invention relates to a hydromassage device with a movable jet or jets. The device comprises a frame and the structure that is arranged as area of support, for instance as reclining surface substantially horizontally, on one side and for receiving the moveable jet or jets from the other side.

In the context of the present invention a hydromassage device shall be understood as comprising all types of devices that provide a kinetic force through the means of a medium onto a body or body part for therapeutic or wellness purposes. The medium is preferably a fluid which can be propelled for instance as a jet towards the body or body part. In a particularly preferred embodiment, the present invention relates to devices employed for physical therapy and physical stimulation through kinetic force, encompassing impulses onto, kneading, working and/or impacting a body or body part with a jet. In a particularly preferred embodiment, the fluid comprises water or essentially consists of water.

Further in the context of the present invention the mat is to be understood as a mat being placed between a source of the kinetic force of the medium and the recipient, i.e. the body or body part. Therefore, a part at least of said kinetic is transmitted onto the body or body part through the mat. In a particularly preferred embodiment, the medium, in particular the fluid, is completely contained and the mat presents a contact surface of a therapeutic device according to the present invention with which the body or body part can be made to receive the kinetic force applied by the medium.

The sensor elements are arranged such that the first and second forces are detectable. The first force is generally created through contact by a patient or person laying, sitting, or standing on the structure; thus, the first force can be considered generally created through gravity or pressure. In other words, the patient or a user at or on the structure creates a certain pressure from one side, for instance from the top. In this example, the pressure of the first force is created by the weight of the user. In general, the first force is created through physical contact between a patient and the structure.

In a particular embodiment the sensor elements enable detection of a position. This can be achieved for instance by the sensor elements comprising position or positioning sensors as these can be used to detect user features, such as body shape or size. With that a body contour recognition and body outline or shape detection can be achieved which can be used for individual user adapted therapeutic applications. Alternatively, or additionally, the position detection can be achieved by a force sensing.

The second force is generally created through pressure to the patient or person laying or sitting on or at the structure. In other words, the user on or at the structure receives pressure forces to parts of the body, usually from the bottom. This pressure of the second force is a jet-pressure force created by a jet, preferably a water jet with a treatment pressure of 0.5 - 4.0 bar. The jet-pressure can be created by nozzles. From such nozzles, which can be moved in the same longitudinal movement and in some embodiments independently or in the same way, water jets are pumped from below against the structure on which the patient or user is lying. Thus, in a particular embodiment, the sensor elements comprise pressure sensors that detect impact forces such as the jet-pressure. Together with the detected body shape or size, the jet-pressure can be controlled and adapted. Further, with the detection of the body shape or size, an individual user can be identified, and, based on a predetermined therapy scheme, a respective program can be applied. This can lead to the nozzles being targeted with a defined jet-pressure to such parts of the body where a treatment is most effective. An intensity can be adjusted and controlled accordingly.

In a particular embodiment, the sensor elements comprise sensors capable of sensing a temperature and/or a temperature change. This can be achieved by temperature sensors, for instance. In a particular embodiment, the temperature sensor comprises a sensing means selected from the group consisting of negative temperature coefficient (NTC) thermistor, resistance temperature detector (RTD), thermocouple and semiconductor-based sensing.

When the sensor elements comprise temperature sensors a temperature can be sensed and measured. This can further support regulation of the temperature by a therapeutic device. The temperature can be adjusted by activating or deactivating one or more heating elements, for instance by means of a control logic and/or processing unit.

For example, a water heating system can be incorporated in a therapeutic device, either as being part of the structure and/or as being part of a medium, such as the fluid. The heating can then for instance be more uniformly dispensed because the heating system is integrated into the structure or mat in an energy-saving manner. This can also help at reducing cooling efforts of the entire device. The entire water in the frame does not need to be heated which saves electrical energy. The heating within the structure or mat is not only more comfortable for the user, the controlling and influencing with the help of the temperature sensors is also faster. That means a desired temperature can be achieved and provided quicker with-out the need to heat massive volumes of water.

With the arrangement of the sensor elements various functions such as position recognition or size recognition of the user can be realized. An automatic adjustment of the travel paths of the jet, pressure recognition and control of the water jet could be realized by means of corresponding sensors in the structure or mat. Also, only certain areas can be heated. Alternatively, various areas or portions might be heated to different temperatures.

The sensor elements can be arranged in different layers. This has the advantage that sensors of the same type are positioned within one layer whereas other sensors are within further layers. It might also be advantageous to arrange different sensors and/or heating elements within the same layer, such that the structure can be kept thin.

If the at least one layer comprises light emitting diodes (LEDs) then light effects can be created that help to guide a patient on the correct positioning. The LEDs can make the application or the device more attractive. The LEDs can be arranged within the same layer as the sensor and/or heating elements thereby reducing the thickness of the structure. However, the LEDs can also be arranged in different layers for production or effect purposes. With the help of the LEDs signals or instructions can be given to the users, e.g. to move or to change position. This might be advantageous if a patient is enjoying music or sounds via a headphone or other sound sources that should not be interrupted by voice or sound signals. The light effects might signal a certain progress or the end of the therapeutic application.

The structure may comprise electrical connections for electrical signals. The signals can be transferred from the structure to a separate or integrated controller. Such a controller unit may process the signals from the various sensors with respect to pressure or temperature. The electrical connections might have different thicknesses. The electrical connections to the heating elements might be thicker to allow a suitable current for the heating purposes.

If the structure comprises different fibres or threads, including optical fibres, desired properties can be achieved, e.g. colour or stability. For example, when the structure consists of a fabric into which the different fibres are woven together in several layers, then corresponding properties such as density, tensile strength, or OF properties can be achieved. In addition, position sensors, pressure sensors, heating elements, LEDs, etc. with the corresponding electrical connections can be integrated into the fabric to create a kind of smart mat. The strength of the mat with certain elastic fibres could be in-creased considerably.

Optical waveguides or optic fibres in the structure could be used for light effects, but also for detection and measurement.

The structure or mat can be arranged between at least a body part of a user and an impacting force. If the structure is thin a direct transfer of the massage or jet forces to and into the body parts is achievable. Different massage types can be provided through the structure or mat, such as parallel massage, mirror image massage, circle massage, or parallel stroke massage.

If the at least one layer is sandwiched between an upper layer and a lower layer, then the one layer is protected by the upper and lower layers. More than three layers might be advantageous, depending on the applications and use.

When the lower layer comprises an elastic waterproof film, the structure is protected and a patient on the structure or support remains dry. If the layer does not leave or leak though any water and possess waterproof properties, the overlaying layers can have electrical connections to lead electrical signals and current in order to function without any short circuit.

According to another aspect a method of forming a structure for therapeutic applications is provided. The method comprises the steps of weaving a plurality of fibres into a fabric and adding sensor elements and heating elements between the fibres to form at least one layer that is affectable or influenceable with a first force from one side and a second force from the other side. The sensor elements can detect different forces.

A further aspect of the invention relates to a non-therapeutic method for operating a hydromassage device comprising the structure with the steps of providing a processing unit with sensor data indicative of a first force affecting a layer from one side and with sensor data indicative of a second force affecting the layer from the other side, providing at least one means for applying the second force to the layer, and adapting the second force reflective of the sensor data indicative of the first force affecting the layer from one side and the sensor data indicative of the second force affecting the layer from the other side.

The means for applying the second force to the layer is preferably a jet and the second force is a jet-pressure force created by the jet, in particular by a water jet with a treatment pressure.

The first force can be created through a physical contact between a patient and the structure.

A body shape or size of a patient can be identified by means of sensor data indicative of a first force, and, based on a predetermined therapy scheme, a respective program can be applied, in particular whereby an adapting the second force comprises a targeting nozzles to parts of the body of the patient based on a body shape or size identified.

The adapting can comprise an adjusting and controlling of an intensity of the second force dependent on the sensor data indicative of the first force affecting the layer from one side and the sensor data indicative of the second force affecting the layer from the other side.

### Brief Description of the Drawings

In order to facilitate better understanding of the present invention, reference is made below to the drawings. These show only exemplary embodiments of the subject matter of the invention.

In the figures and the associated description, identical or functionally analogous parts or elements are provided with the same reference numerals. The same or identical signs or symbols denote the same or identical elements.
- Fig. 1: shows a schematic representation of a structure.
- Fig. 2: shows a further schematic representation of the structure with impacting forces.
- Fig. 3: shows a further schematic representation of the structure with LEDs.
- Fig. 4: shows a schematic representation of another structure with multiple layers.
- Fig. 5: shows a schematic representation of a further structure with multiple layers.
- Fig. 6: shows a schematic representation of a hydromassage device.
- Fig. 7: shows a schematic representation of a hydromassage device impacting forces from both sides.
- Fig. 8: shows a schematic representation of a structure with electrical connections and a central control or processing unit.
- Fig. 9: shows a schematic representation of a hydromassage device with a patient on the structure.
- Fig. 10: shows a schematic representation of a massage device with a patient on the structu re.

### Detailed Description

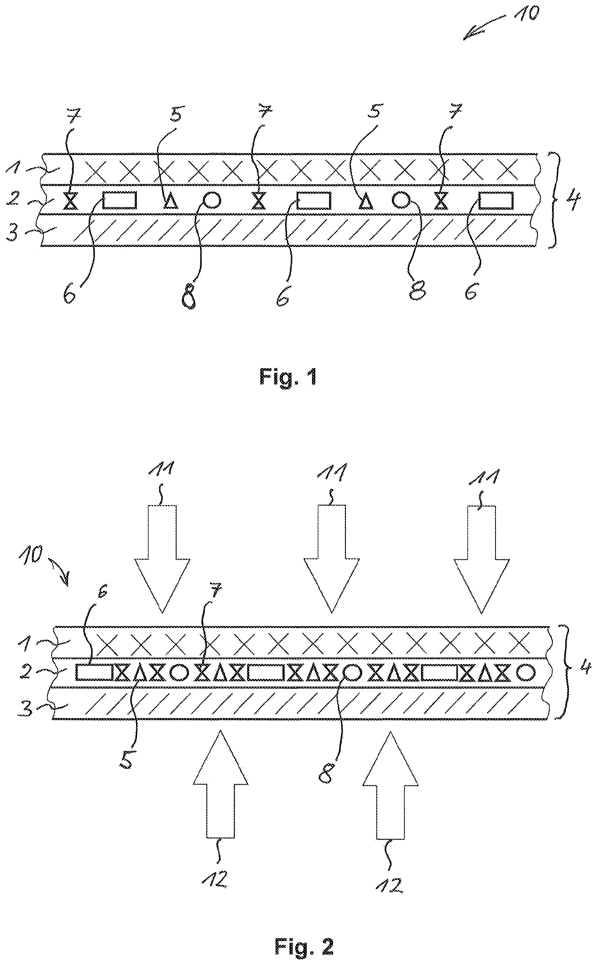

Figure 1 shows a cross section of a structure 10 for therapeutic applications. The structure 10 comprises an upper layer 1, a middle layer 2, and a lower layer 3. The structure 10 is formed as a mat 4 that has certain characteristics. The three-layer woven mat 4 contains various fibres in different layers. The upper layer 1 is a resist or protective coating. The middle layer 2 comprises various sensor elements 5, 6, 7. In the same layer are arranged position sensors 5, pressure sensors 6, temperature sensors 7, and heating elements 8. All sensors and elements have electrical or other connections (not shown). The lower layer 3 comprises an insulating coating. As illustrate in the figure the middle layer 2 is sandwiched between the upper layer 1 and the lower layer 3.

In an embodiment of the present invention different fibres, also referred to as threads, are woven together in the several layers 1, 2, 3 in order to achieve respective properties, e.g. to achieve tightness, tensile strength, or a respective structure or colour. Advantageously, the position sensors 5, the pressure sensors 6, and the heating elements 8 are integrated into the fabric that results in the mat 4 with the appropriate connections in order to create a kind of smart mat. Each layer 1, 2, 3 may have a certain thickness, preferably in the range of millimetres to some centimetres.

Figure 2 shows a further embodiment of structure 10 with the upper layer 1, the middle layer 2, and the lower layer 3, but with a modified arrangement of the elements, i.e. sensor elements 5, 6, 7 and heating elements 8. The structure 10 forms the mat 4. The middle layer 2 comprises the various sensor elements 5, 6, 7 and the heating elements 8.

Forces 11, 12 may act from both sides on the mat 4 and thus particularly on the middle layer 2. From the top first forces 11 act on the middle layer 2. From the bottom second forces 12 act on the middle layer 2. It is noted that the forces 11, 12 influence and act on all layers of the mat 4, but the detection of the forces takes place by the sensor elements 5, 6 in the middle layer 2. The sensor elements 5, 6 are arranged such that the first and second forces 11, 12 are detectable. The arrangement of the sensor elements 5, 6, 7 and heating elements 8 may follow a certain pattern. It is particularly favourable to arrange more position sensors 5 in areas where a patient is potentially reclined, whereas areas, e.g. at the edges or corners can be omitted or the density of the position sensors 5 can be reduced. Electrical signals are sent or transmitted via electrical connections to and from a control unit or central processing unit (not shown in Fig. 2).

The first force 11 is created through gravity by a patient or person laying or sitting on the mat 4. In general, a user on the mat 4 creates a certain pressure from the top. This pressure is created by weight of the user.

By measuring the pressure of the first forces 11 the exact position of a user can be determined. That means, that user features like the size or body contour of the user can be determined, and an application to the user can be applied and adapted accordingly. Individual user adapted therapeutic applications can be offered which are tailor made for specific needs or desires of a user.

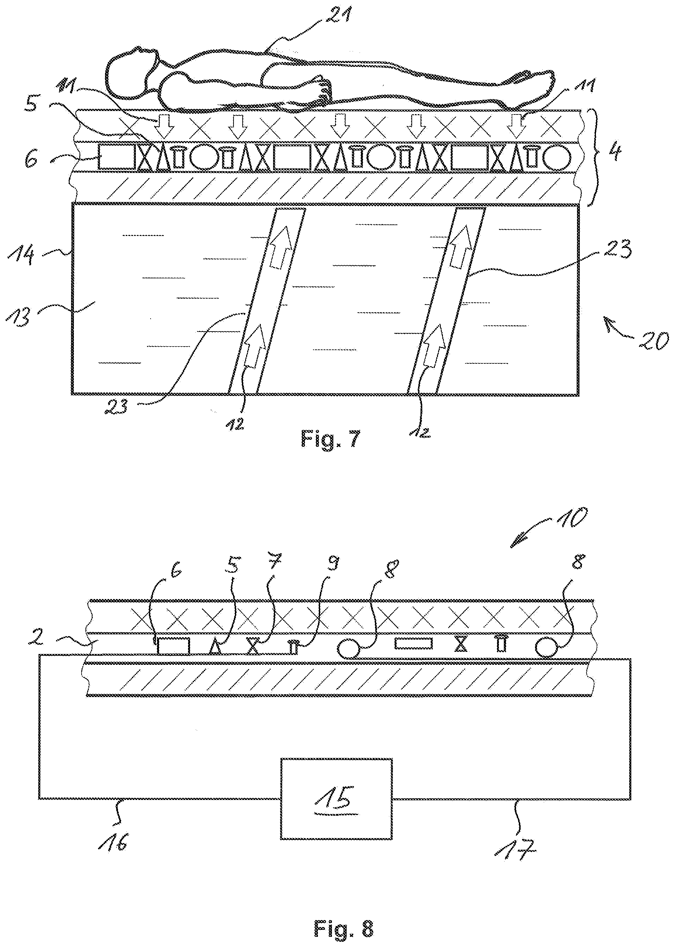

The second force 12 results from a massage instrument or device with a moving drum or a jet. Such impact forces are detected by the pressure sensors 6. Electrical signals from the pressure sensors 6 are sent via the electrical connections to the control unit or central processing unit (shown in Fig. 8). In general, the second force 12 is a force that acts from the outside on the mat 4 and consequently to a patient on the mat 4. The second force or forces 12 are created and applied for the therapeutic effect, e.g. massage effect.

The temperature sensors 7 help to determine and measure the temperature at the middle layer 2. The temperature sensors 7 send electrical signals via the electrical connections to the control unit or central processing unit for processing. The heating elements 8 are heated accordingly, i.e., based on the detected and measured temperature the heating elements 8 are activated to heat up the mat 4.

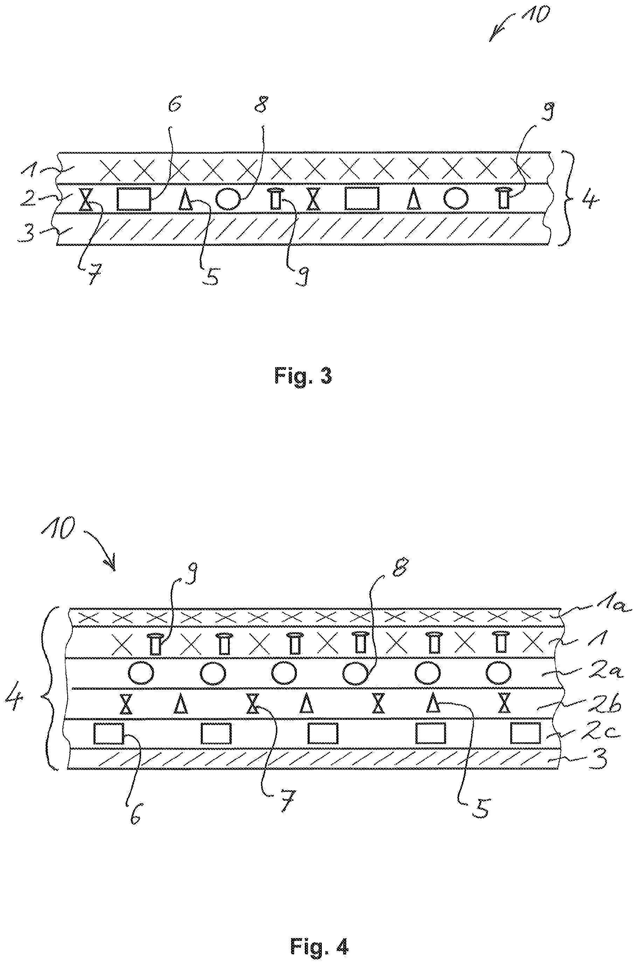

Figure 3 shows a further embodiment of structure 10 with the upper layer 1, the middle layer 2, and the lower layer 3. The structure 10 forms the mat 4. The middle layer 2 comprises the various sensor elements 5, 6, 7, the heating elements 8, and light emitting diodes 9. The LEDs 9 are arranged within the middle layer 2 but could also be arranged separately on or within another layer. All elements have connections to the outside (not shown).

The LEDs 9 create light effects which guide a patient on the mat 4 for exact positioning. The LEDs 9 can also make an application or the device more attractive. The LEDs 9 can for example guide a user on the first lay down or the correct positioning of an arm or leg. By indications of LED signals, the users can be given instructions, e.g. where to move an arm or when an application has finished.

Figure 4 shows a further embodiment of structure 10 with multiple layers. The structure 10 forms the mat 4 with at least five layers. The structure 10 comprises a top layer 1a with a protective coating, the upper layer 1, a third layer 2a, a fourth layer 2b, a fifth layer 2c and the lower layer 3. The LEDs 9 are here arranged at the upper layer 1 in order to allow a direct light emission towards the reclining surface though the top layer 1 with the protective coating. The third layer 2a comprises the heating elements 8. The fourth layer 2b comprises two sensor elements 5, 7, namely the position sensors 5 and the temperature sensors 7. Underneath, the pressure sensors 6 are arranged within the fifth layer 2c. Finally, the lower layer 3 is located at the bottom of the stack. In general, any kind of combination of the various elements and layers is possible. It might be advantageous to have certain sensors and/or elements in one layer whereas other sensors and/or elements are in a separate layer. An advanced manufacturing process may even allow to arrange all elements in one layer such that a thin, but robust mat 4 can be provided.

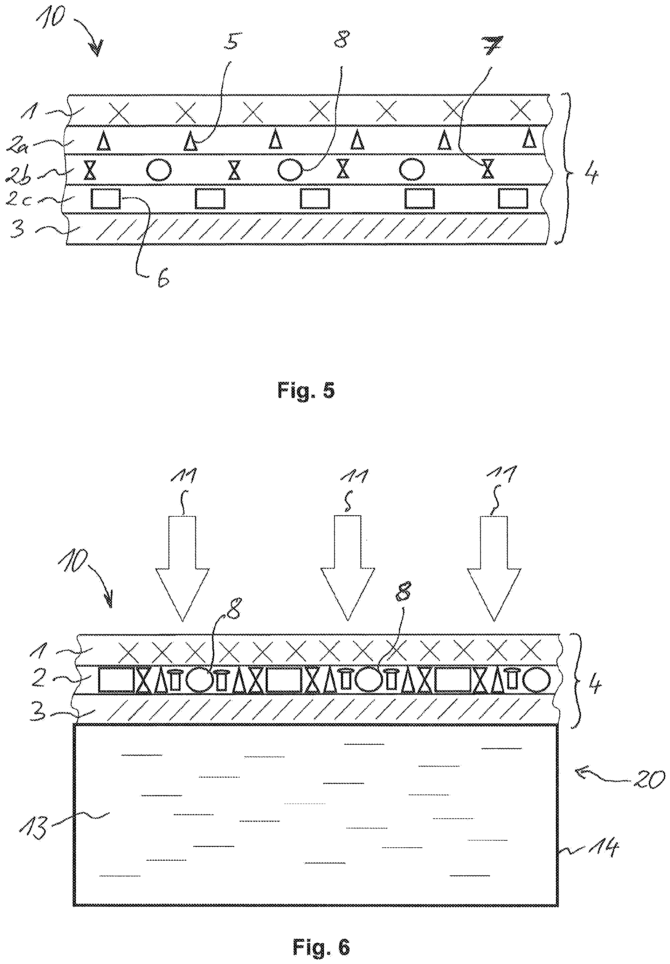

Figure 5 shows yet a further embodiment of structure 10 with multiple layers. The structure 10 with five layers forming the mat 4. The structure 10 comprises the upper layer 1, a second layer 2a, a third layer 2b, a fourth layer 2c and the lower layer 3 as the fifth layer. No LEDs are integrated. The upper layer 1 is a protection layer to the surface on which a patient will be positioned. The second layer 2a comprises the position sensors 5 for precise detection of the patient. The third layer 2b comprises two components 7, 8; the temperature sensors 7 and the heating elements 8 are arranged within the same layer. It might be advantageous for the manufacturing process to have the temperature sensor 7 and the heating elements 8 in the same layer. Below the third layer 2b, the pressure sensors 6 are arranged within the fourth layer 2c. The lower layer 3 consists of a water-resistant film that is explained in more detail below.

Figure 6 shows a cross-section of a hydromassage device 20. In this embodiment the structure 10 comprises the upper layer 1, the middle layer 2, and the lower layer 3. Impacting first forces 11 are created from the top by a patient (not shown) on the mat 4. The lower layer 3 of the mat 4 is in direct contact with water 13 that is contained in a frame 14. The structure 10 is substantially horizontal as the mat 4 is on top of the water 13. The lower layer 3 has an insulating coating. This material is waterproof in order to not allow the other layers to become wet or effected by the water 13. The water 13 is arranged in a tank that is located within the frame 14. It is particularly advantageous that the water 13 in the tank does not need to be heated for comfort. This reduces energy. The heating is enabled directly within the mat 4 through the heating elements 8 to about 20 to 40 degrees Celsius.

The tank is filled with over 300 litres of water. The pressure strength, massage type and areas can be individually controlled for each treatment by the patient, but also controlled fully automatically through a control unit as described with reference to Figure 8 below.

The hydromassage device 20 provides various massage types such as parallel massage, mirror image massage, circle massage, or parallel stroke massage. Further, massage areas are selectable or applied through detection such as shoulder and/or neck massage, whole body, back, lumbar area, legs, etc. The duration of an application is usually in a range up to 45 minutes. The intensities of the pressure can be individually selected, but with the help of the pressure sensor 6 the pressure can be controlled in an optimized way.

Figure 7 shows the hydromassage device 20 of Fig. 6 with two massage jets from the bottom exerting the second forces 12. Generally, from movable nozzles (not shown), the water jets 21 are pumped from below against the mat 4 on which a patient 21 is lying. The second forces 12 are detected by the pressure sensors 6. Electrical signals from the sensors 5, 6 are sent via electrical connections to the control unit or central processing unit. A treatment with defined pressure against areas is performed in response to the determined position of the patient through the position sensors 5.

Figure 8 illustrates the structure 10 with the middle layer 2 and electrical connections 16, 17 to a control unit or central processing unit 15. First electrical connections 16 connect the sensor elements 5, 6, 7 to the central processing unit 15. The LEDs 9 are also connected through the first electrical connections 16 to the central processing unit 15. Second electrical connections 17 connect from the control unit 15 to the heating elements 8. The second electrical connections 16 are adapted to the current or power needs of the heating elements 8. That means in practice that the second electrical connections 17are thicker than the first electrical connections 16.

The sensor elements 5, 6, 7 communicate with the central processing unit 15. The central processing unit 15 which can be connected to a network and further to the Internet enables various applications and treatments. As indicated above different massage types can be provided and various massage areas be addressed.

The position sensors 5 detect the position of user or certain parts of the body which require a treatment. The position sensors 5 might be adapted to detect the hardness of tissue or muscles. In response to that specific applications to certain areas or muscles with defined types and pressure are applied, all processed and controlled through the central processing unit 15. Software programs with specific code can be applied. The use of self-learning or adapting programs will result in more optimized applications for the benefit of the patient who receives a treatment that is individually most suitable and not a standard program.

The pressure sensors 6 detect the jet pressure from below. The jet pressure, i.e. the intensity, and the movement of the jet are controlled by the control or central processing unit 15. Based on the detected body contour of the patient a treatment program is applied that is most effective to the patient. With the help of the pressure sensors 6 the pressure intensity can be controlled in an optimized way.

The position sensors 5 and pressure sensors 6 can be realized by piezoelectric sensors. Such piezoelectric sensors use the piezoelectric effect to measure changes in pressure, acceleration, strain, or force by converting them to an electrical charge. Piezoelectric sensors do not require any power to continuously monitor an environment. They offer stable and repeatable accurate electrical output. With such sensors also the weight of a patient can be determined. Based on that, the jet pressure might be adapted.

The temperature sensors 7 help to determine and measure the temperature at the middle layer 2. The temperature sensors 7 deliver an electrical signal as a measure of the temperature and could be arranged within any other layer but are arranged where the temperature is detectable in an optimal way. The temperature sensors 7 send electrical signals via the first electrical connections 16 to the control or central processing unit 15 for processing. The heating elements 8 are heated through respective power applied to the second electrical connections 17. The heating and cooling process is controlled and adapted by the control or central processing unit 15. That means based on the detected and measured temperature the heating elements 8 are activated to heat up the structure 10.

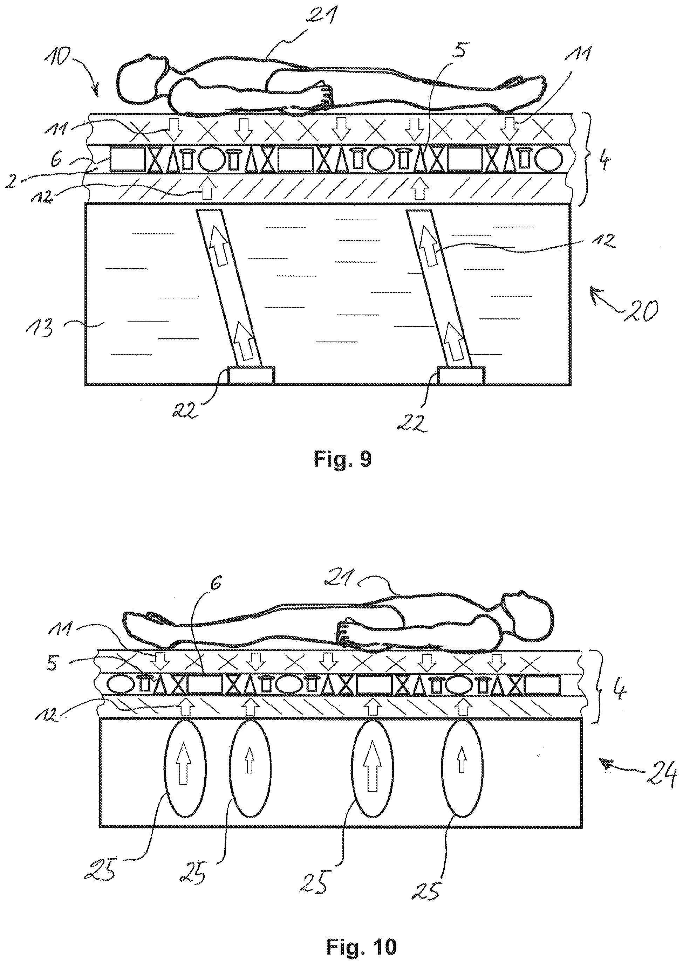

Figure 9 shows a schematic representation of a hydromassage or jet device 20 with a user 21 on the structure 10. As illustrated in the figure the user or patient 21 reclines on the mat 4 and enjoys a therapeutic application in the form of a water jet massage through impacting forces, the second forces 12, created through nozzles 22. The patient 21 himself creates certain pressure from the top whereby the resulting first forces 11 are detected and measured by the position sensors 5. The structure 10 is arranged between the body of the patient or user 21 and the impacting force 12. The pressure sensors 6 detect the jet pressure as the second forces 12 from below. The jet pressure, the movement of the jet, and the temperature are controlled by the control or central processing unit 15 (not shown in the figure). Based on a program and the detected body contour of the patient a treatment is applied that is most effective for the patient.

As indicated above the structure 10 can be a fabric into which different fibres are woven. The structure 10 for therapeutic applications is produced by weaving a plurality of fibres into the fabric, adding sensor elements 5, 6, 7 and heating elements 8 between the fibres to form at least one layer 2. The layer 2 is affectable with the first force 11 from one side, usually form the top, and the second force 12 from the other side, usually from the bottom. The mat 4 can be realized as an e-textile combining electronic devices such as the sensor elements 5, 6, 7 and heating elements 8 with textiles. The electronics with active and passive elements are integrated directly into the textile substrates. The conducting textile weaves are connected to the control or central processing unit 15. Printing techniques could be applied to create the sensor elements 5, 6, 7 and heating elements 8.

Figure 10 shows a schematic representation of a massage device 24 with a patient 21 on the structure 10. The massage device 24 comprises moving drums 25. As illustrated in the figure the patient 21 in positioned on the mat 4 horizontally and enjoys a therapeutic application in the form of a massage through impacting forces, the second forces 12, created through the moving drums 25. The patient 21 himself creates certain pressure from the top whereby the resulting first forces 11 are detected and measured by the position sensors 5. The structure 10 is arranged between the body of the patient or user 21 and the impacting force 12. The pressure sensors 6 detect the pressure as the second forces 12 from the moving drums 25. The pressure, the movement of the drums 25, and the temperature are controlled by the control or central processing unit 15 (not shown in the figure). Based on a program and the detected body contour of the patient a treatment is applied that is most effective for the patient. The arrangement of the mat 4 is flexible, depending form where the second forces 12 are applied. The means the mat 4 can also be applied if the patient 21 is sitting, standing, or in an inclined position.

The embodiments described and shown can be combined with each other and are intended to illustrate one or more ways to practice and implement the present disclosure. It is further noted that the foregoing description of the specific embodiments of the subject matter disclosed herein has been presented for purposes of illustration and description and is not intended to limit the scope of the subject matter set forth herein. It is fully contemplated that other various embodiments, modifications and applications will become apparent to those of ordinary skill in the art from the foregoing description and accompanying drawings. Thus, such other embodiments, modifications, and applications are intended to fall within the scope of the following appended claims. Further, those of ordinary skill in the art will appreciate that the embodiments, modifications, and applications that have been described herein are in the context of particular environment, and the subject matter set forth herein is not limited thereto, but can be beneficially applied in any number of other manners, environments and purposes. Accordingly, the claims set forth below should be construed in view of the full breadth of the novel features and techniques as disclosed herein.

### List of references

- 1: upper layer
- 2: middle layer
- 3: lower layer
- 4: mat
- 5, 6, 7: sensor elements
5 position sensors
6 pressure sensors
7 temperature sensors
- 8: heating elements
- 9: light emitting diodes (LEDs)
- 10: structure
- 11: first force
- 12: second force
- 13: water
- 14: frame
- 15: control or central processing unit
- 16: first electrical connections
- 17: second electrical connections
- 20: hydromassage device
- 21: patient or user
- 22: nozzles
- 23: water jet
- 24: massage device
- 25: moving drum

## Claims

1. A hydromassage device with a movable jet comprising
a frame (14) and
a structure (10) for therapeutic applications, the structure comprising:
at least one layer (2) with sensor elements (5, 6, 7), wherein, in use, a first force (11) affects
the layer (2) from one side and a second force (12) affects the layer (2) from the other side,
**characterized in that,**
the sensor elements (5, 6, 7) are arranged to detect the first and second (11, 12) forces, and wherein
the at least one layer (2) is sandwiched between an upper layer (1) and a lower layer (3), and
the structure (10) being arranged substantially horizontally as reclining surface on one side and for receiving the moveable jet from the other side.

2. The hydromassage device of claim 1, wherein the layer further comprises heating elements (8).

3. The hydromassage device according to any one of claims 1 or 2, wherein the sensor elements (5, 6, 7) comprise position sensors (5) for detecting user features.

4. The hydromassage device according to any preceding claim, wherein the sensor elements (5, 6) comprise pressure sensors (6) for detecting impact forces.

5. The hydromassage device according to any preceding claim, wherein the sensor elements (7) comprise temperature sensors (7) for determining a temperature.

6. The hydromassage device according to any preceding claim, wherein the sensor elements (5, 6, 7) are arranged in different layers.

7. The hydromassage device according to any preceding claim, wherein the at least one layer (2) comprises light emitting diodes for light effects.

8. The hydromassage device according to any preceding claim, the structure (10) further comprising electrical connections for electrical signals.

9. The hydromassage device according to any preceding claim, the structure further comprising different fibres.

10. The hydromassage device according to claim 9, the structure (10) consisting of a fabric into which the different fibres are woven.

11. The hydromassage device according to any preceding claim, the structure being arranged between at least a body part of a user and an impacting force.

12. The hydromassage device according to any preceding claim, wherein the lower layer (3) comprises an elastic waterproof film.

13. A non-therapeutic method for operating the hydromassage device according to any of the claims 1 to 12, comprising the steps of:
a. providing a processing unit (15) with sensor data indicative of a first force (11) affecting a layer (2) from one side and with sensor data indicative of a second force (12) affecting the layer (2) from the other side;
b. providing at least one means for applying the second force (12) to the layer (2); and
c. adapting the second force (12) reflective of the sensor data indicative of the first force (11) affecting the layer (2) from one side and the sensor data indicative of the second force (12) affecting the layer (2) from the other side.

14. The non-therapeutic method according to claim 13, whereby an adapting comprises an adjusting and controlling of an intensity of the second force (12) dependent on the sensor data indicative of the first force (11) affecting the layer (2) from one side and the sensor data indicative of the second force (12) affecting the layer (2) from the other side.

15. The non-therapeutic method according to any one of claims 13 to 14, whereby the structure comprises temperature sensors (7) for determining a temperature, the method comprising the further step:
a. Sensing a temperature by means of at least one temperature sensor (7) in the structure, and
b. Controlling the temperature by means of heating elements (8) controlled by the central processing unit (15).

## Patentansprüche

1. Hydromassagevorrichtung mit einem bewegbaren Strahl, umfassend
ein Gestell (14), und
eine Struktur (10) für therapeutische Anwendungen, wobei die Struktur umfasst:
mindestens eine Schicht (2) mit Sensorelementen (5, 6, 7), wobei bei Gebrauch eine erste Kraft (11) von einer Seite aus auf die Schicht (2) einwirkt und eine zweite Kraft (12) von der anderen Seite aus auf die Schicht (2) einwirkt,
**dadurch gekennzeichnet, dass**
die Sensorelemente (5, 6, 7) angeordnet sind, um die erste und die zweite (11, 12) Kraft zu detektieren, und wobei
die mindestens eine Schicht (2) zwischen einer oberen Schicht (1) und einer unteren Schicht (3) eingeschoben ist, und
die Struktur (10) auf einer Seite im Wesentlichen waagerecht als Liegefläche und von der anderen Seite zum Empfangen des bewegbaren Strahls angeordnet ist.

2. Hydromassagevorrichtung nach Anspruch 1, wobei die Schicht ferner Heizelemente (8) umfasst.

3. Hydromassagevorrichtung nach einem der Ansprüche 1 oder 2, wobei die Sensorelemente (5, 6, 7) Positionssensoren (5) umfassen, um die Merkmale des Benutzers zu detektieren.

4. Hydromassagevorrichtung nach einem der vorhergehenden Ansprüche, wobei die Sensorelemente (5, 6) Drucksensoren (6) umfassen, um Aufprallkräfte zu detektieren.

5. Hydromassagevorrichtung nach einem der vorhergehenden Ansprüche, wobei die Sensorelemente (7) Temperatursensoren (7) umfassen, um eine Temperatur zu bestimmen.

6. Hydromassagevorrichtung nach einem der vorhergehenden Ansprüche, wobei die Sensorelemente (5, 6, 7) in verschiedenen Schichten angeordnet sind.

7. Hydromassagevorrichtung nach einem der vorhergehenden Ansprüche, wobei die mindestens eine Schicht (2) Leuchtdioden für Lichteffekte umfasst.

8. Hydromassagevorrichtung nach einem der vorhergehenden Ansprüche, wobei die Struktur (10) ferner elektrische Anschlüsse für elektrische Signale umfasst.

9. Hydromassagevorrichtung nach einem der vorhergehenden Ansprüche, wobei die Struktur ferner verschiedene Fasern umfasst.

10. Hydromassagevorrichtung nach Anspruch 9, wobei die Struktur (10) aus einem Gewebe besteht, in das die verschiedenen Fasern eingewebt sind.

11. Hydromassagevorrichtung nach einem der vorhergehenden Ansprüche, wobei die Struktur zwischen mindestens einem Körperteil eines Benutzers und einer Aufprallkraft angeordnet ist.

12. Hydromassagevorrichtung nach einem der vorhergehenden Ansprüche, wobei die untere Schicht (3) eine elastische, wasserdichte Folie umfasst.

13. Nicht therapeutisches Verfahren zum Betreiben der Hydromassagevorrichtung nach einem der Ansprüche 1 bis 12, umfassend folgende Schritte:
a) Bereitstellen für eine Verarbeitungseinheit (15) von Sensordaten, die bezeichnend sind für eine erste Kraft (11), die von einer Seite aus auf eine Schicht (2) einwirkt, und von Sensordaten, die bezeichnend sind für eine zweite Kraft (12), die von der anderen Seite aus auf die Schicht (2) einwirkt;
b) Bereitstellen mindestens eines Mittels zum Ausüben der zweiten Kraft (12) auf die Schicht (2); und
c) Anpassen der zweiten Kraft (12) entsprechend der Sensordaten, welche bezeichnend sind für die erste Kraft (11), die von einer Seite aus auf die Schicht (2) einwirkt, und der Sensordaten, die bezeichnend sind für die zweite Kraft (12), die von der anderen Seite aus auf die Schicht (2) einwirkt.

14. Nicht therapeutisches Verfahren nach Anspruch 13, wobei ein Anpassen eine Einstellung und Steuerung einer Intensität der zweiten Kraft (12) in Abhängigkeit von den Sensordaten, die bezeichnend sind für die erste Kraft (11), die von einer Seite aus auf die Schicht (2) einwirkt, und den Sensordaten, die bezeichnend sind für die zweite Kraft (12), die von der anderen Seite aus auf die Schicht (2) einwirkt, umfasst.

15. Nicht therapeutisches Verfahren nach einem der Ansprüche 13 bis 14, wobei die Struktur Temperatursensoren (7) umfasst, um eine Temperatur zu bestimmen, wobei das Verfahren die folgenden weiteren Schritte umfasst:
a) Erfassen einer Temperatur anhand mindestens eines Temperatursensors (7) in der Struktur, und
b) Steuern der Temperatur anhand von Heizelementen (8), die von der zentralen Verarbeitungseinheit (15) gesteuert werden.

## Revendications

1. Dispositif d'hydromassage avec un jet mobile, comprenant
un cadre (14), et
une structure (10) pour des applications thérapeutiques, la structure comprenant :
au moins une couche (2) avec des éléments capteurs (5, 6, 7), dans lequel en cours d'utilisation, une première force (11) affecte la couche (2) depuis un côté, et une seconde force (12) affecte la couche (2) depuis l'autre côté,
**caractérisé en ce que**
les éléments capteurs (5, 6, 7) sont agencés pour détecter la première et la seconde (11, 12) force, et dans lequel
ladite au moins une couche (2) est prise en sandwich entre une couche supérieure (1) et une couche inférieure (3), et
la structure (10) est agencée de manière substantiellement horizontale comme une surface de couchage sur un côté et pour recevoir le jet mobile depuis l'autre côté.

2. Dispositif d'hydromassage selon la revendication 1, dans lequel la couche comprend en outre des éléments chauffants (8).

3. Dispositif d'hydromassage selon l'une quelconque des revendications 1 ou 2, dans lequel les éléments capteurs (5, 6, 7) comprennent des capteurs de position (5) pour détecter les particularités de l'utilisateur.

4. Dispositif d'hydromassage selon l'une quelconque des revendications précédentes, dans lequel les éléments capteurs (5, 6) comprennent des capteurs de pression (6) pour détecter des forces d'impact.

5. Dispositif d'hydromassage selon l'une quelconque des revendications précédentes, dans lequel les éléments capteurs (7) comprennent des capteurs de température (7) pour déterminer une température.

6. Dispositif d'hydromassage selon l'une quelconque des revendications précédentes, dans lequel les éléments capteurs (5, 6, 7) sont agencés dans différentes couches.

7. Dispositif d'hydromassage selon l'une quelconque des revendications précédentes, dans lequel ladite au moins une couche (2) comprend des diodes électroluminescentes pour des effets de lumière.

8. Dispositif d'hydromassage selon l'une quelconque des revendications précédentes, la structure (10) comprenant en outre des connexions électriques pour des signaux électriques.

9. Dispositif d'hydromassage selon l'une quelconque des revendications précédentes, la structure comprenant en outre différentes fibres.

10. Dispositif d'hydromassage selon la revendication 9, la structure (10) étant composée d'un tissu dans lequel différentes fibres sont tissées.

11. Dispositif d'hydromassage selon l'une quelconque des revendications précédentes, la structure étant agencée entre au moins une partie du corps d'un utilisateur et une force d'impact.

12. Dispositif d'hydromassage selon l'une quelconque des revendications précédentes, dans lequel la couche inférieure (3) comprend un film imperméable élastique.

13. Procédé non thérapeutique pour faire fonctionner le dispositif d'hydromassage selon l'une quelconque des revendications 1 à 12, comprenant les étapes consistant à :
a) fournir à une unité de traitement (15) des données de capteur indiquant une première force (11) affectant une couche (2) depuis un côté et des données de capteur indiquant une seconde force (12) affectant la couche (2) depuis l'autre côté ;
b) fournir au moins un moyen pour appliquer la seconde force (12) à la couche (2) ; et
c) adapter la seconde force (12) reflétant les données de capteur indiquant la première force (11) affectant la couche (2) depuis un côté et les données de capteur indiquant la seconde force (12) affectant la couche (2) depuis l'autre côté.

14. Procédé non thérapeutique selon la revendication 13, selon lequel une adaptation comprend un ajustement et une commande d'une intensité de la seconde force (12) en fonction des données de capteur indiquant la première force (11) affectant la couche (2) depuis un côté et des données de capteur indiquant la seconde force (12) affectant la couche (2) depuis l'autre côté.

15. Procédé non thérapeutique selon l'une quelconque des revendications 13 à 14, selon lequel la structure comprend des capteurs de température (7) pour déterminer une température, le procédé comprenant les étapes supplémentaires consistant à :
a) capter une température au moyen d'au moins un capteur de température (7) dans la structure, et
b) commander la température au moyen d'éléments chauffants (8) commandés par l'unité centrale (15).
